# EUROPEAN PATENT APPLICATION

(11) **EP 1 749 503 A1**
(43) Date of publication of application: **07.02.2007**
(21) Application number: 05425573.2
(22) Date of filing: 02.08.2005
(51) Int. Cl.: A61F 2/00

(54) **Tissue prosthesis**

(71) Applicant: Gandini, Marco, 10040 Almese (Torino) (IT)
(72) Inventor: Gandini, Marco, 10040 Almese (Torino) (IT)
(74) Representative: Freyria Fava, Cristina

(57) **Abstract**

The invention concerns a tissue prosthesis (1) consisting of two layers of material that extend in a substantially coplanar fashion with a perimetral zone (90) in which the two layers are distanced from one another and a central zone (95) in which the two layers are adjacent to one another, and also comprising within the central zone at least one zone (91) in which the two layers are distanced from one another. Cells or drugs such as NO donors or chomotactic substances might be incorporated.

## Description

The present invention concerns tissue prostheses to repair and reconstruct tissue, particularly soft tissues.

The tissue prostheses for soft tissue repair, or "meshes", available on the market today and in particular those for hernias, generally consist of two layers of material adjacent/connected to one another over the entire surface of the mesh. The external layer is in direct contact with the flaps of the tissue to be repaired, and the internal layer is in direct contact with the internal organs. In particular, the external layer generally presents a coarse weave, or in any case a surface that is suitable for adhesion of fibroblasts that - through their reproduction -- reinstate the correct cellular structure of the tissue, while the internal layer is smoother since its structure is intended to prevent the mesh from adhering to the internal organs.

The present invention concerns an innovative tissue prosthesis that can reduce the risk of adherences forming at suture points used to fix it to the abdominal wall.

The present invention concerns an innovative tissue prosthesis able to reduce the mesh dimensions to those of the defect to be repaired without thereby reducing adaptation to the morphological characteristics of the defect requiring treatment.

Another purpose of the present invention is to provide a tissue prosthesis that further facilitates the procedure of suturing the mesh to the tissue undergoing repair.

A further purpose of the present invention is that of producing a tissue prosthesis that can act as a vehicle for pharmacological, tissue or cellular agents able to improve the healing of the tissue undergoing repair.

In accordance with the present invention, these goals are achieved through the solutions specifically expressed in the claims below. The claims form an integral part of the technical instruction provided here in relation to the invention.

The invention concerns a tissue prosthesis consisting of two layers of material that extend substantially in a coplanar fashion, with a perimeter zone in which the two layers are distanced from one another and a central zone in which the two layers are adjacent to one another, and also including within the central zone at least one zone in which the two layers are distanced from one another.

The invention will now be described in detail, purely as a non-restrictive example, with reference to the attached figures, in which:
-- figure 1 illustrates a positioning diagram of a tissue prosthesis to repair a hernia;
-- figures 2, 3, 4, 6 and 7 represent perspective views of different embodiments of the tissue prosthesis subject of the present invention;
-- figure 5 is a section along the axis B-B' of the form illustrated in figure 2.

Henceforth, all references to the external layer 2 and the internal layer 3 are to be understood purely as an example of the present invention and are in no way restrictive in general terms.

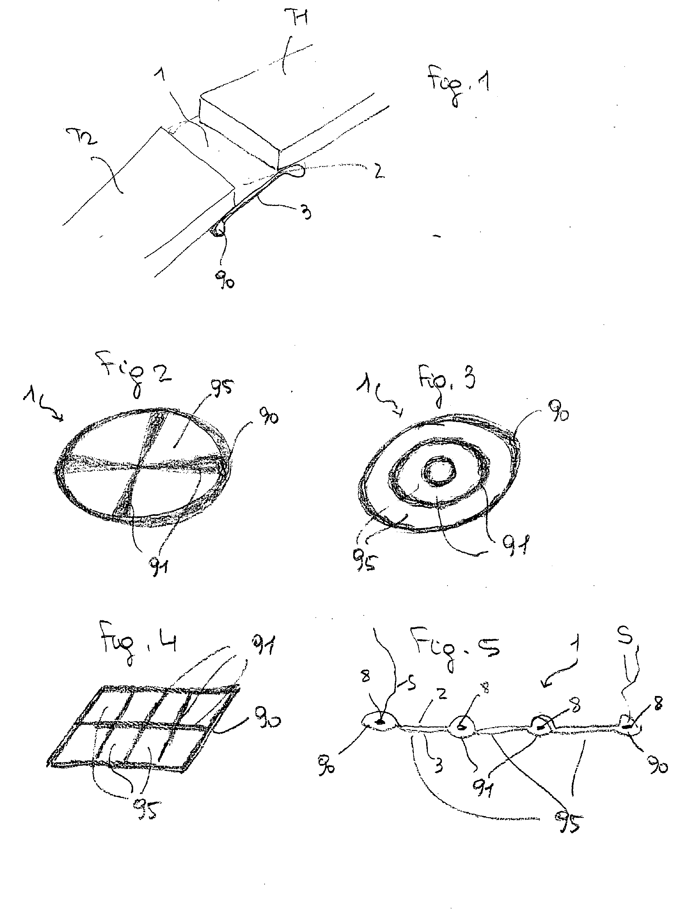

With reference to figure 1, the tissue prosthesis 1 is positioned for example, within the abdominal cavity to repair an abdominal hernia caused by the separation of two tissue flaps T1 and T2. The prosthesis 1 presents its external 2 and internal 3 layers respectively in contact with flaps T1 and T2 of the tissue to be repaired, and with the internal organs of the abdominal cavity, the layers being adjacent to one another in the central zone indicated by reference 95, and distanced one from another in a peripheral or perimetral zone 90.

The tissue prosthesis subject of the present invention is characterised by the presence in the central area 95 defined by the perimeter zone 90 of at least one zone 91 in which the two layers 2 and 3 are distanced from one another, in other words it is characterised by portions 95 of adjacency separated from one another by portions 91 in which the two layers 2 and 3 are distanced from one another.

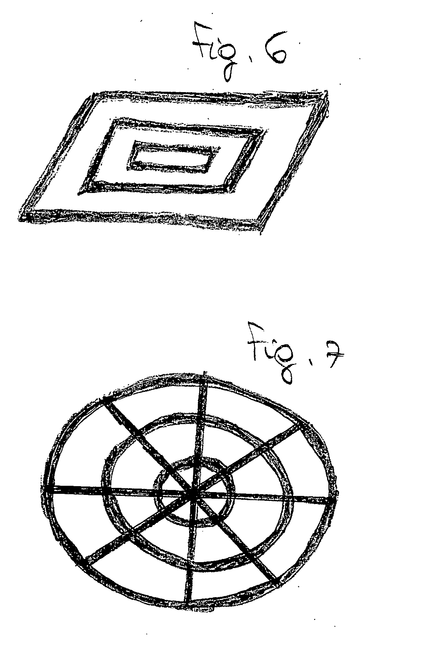

The meshes subject of the present invention therefore present a sequence of alternating solid and hollow zones, as is exemplified in figures 2, 3 and 4, in which the portions 91 in which the two layers are distanced, may take on structures like spokes, ring-shaped, or as a grid.

The structures like spokes, in concentric rings, or as a grid, illustrated in figures 2, 3 and 4, enable the dimensions of the mesh to be modified so that they come as close as possible to the dimensions of the defect to be repaired, without thereby modifying the characteristics of the mesh itself.

Figure 5 represents a cross-section of the tissue prosthesis subject of the present invention. The external layer 2 is adjacent to the internal layer 3 in zones 95; in zones 90 and 91, the two layers 2 and 3 are distanced from one another, leaving a hollow space. This configuration enables suturing or anchorage devices to be positioned within zones 90 and 91 in order to connect the mesh to tissue flaps T1 and T2 undergoing repair and to protect the traditional suture threads which only pass through the external layer 2.

Positioning of suture anchors within zone 91, in which the two layers of the mesh are distanced from one another, facilitates suturing the tissue defect and, in particular, enables the suture anchors 8 to be placed not only on the edges of the defect to be repaired but also in the central area of the defect, improving reduction of the opening.

Furthermore, this particular conformation of zones 90 and 91 avoids suture anchors and suturing systems in general having to be exposed within the abdominal cavity and thus their involvement in the formation of undesirable tissue adherences.

Special holes can optionally be made in layer 2 of the mesh in correspondence with zones 90 and 91, to facilitate introduction of the suture anchors.

A further advantage of the tissue prosthesis subject of the present invention is given by the possibility of incorporating, in zones 90 and 91, or on the surface of one or both layers 2 and 3: i) islands of tissue, for example connective tissue of the ligamentous or tendinous type, from the linea alba, etc., obtained from explantation, autologous or heterologous grafting, including from different species (in particular the pig); ii) differentiated cells or stem cells; iii) cell cultures in general and fibroblast cultures in particular; iv) drugs; or v) devices able to improve and encourage healing of the tissue undergoing repair to which the said tissue prosthesis is applied.

In relation to the incorporation of drugs on the mesh of the present invention it is clear that such drugs can be fixed employing any known physical or chemical method and may also be associated to gradual or delayed release systems.

Among the drugs that may be used to advantage in the present invention, the following may be quoted purely as a non-restrictive example: NO donors, such as for example S-nitrosothiols, detanonoate, SIN-1, molsidomine, isosorbide mono- and di-nitrate, eritrile and pentaeritrile tetranitrate, tenitramine and, in particular, glyceryl trinitrate.

Among other substances that can advantageously be applied onto the tissue prosthesis object of this invention may be quoted, purely as a non-restrictive example, chemotactic substances for fibroblasts and in particular elastin, amino acids, polyamino acid complexes, hyaluronic acid. Among amino acids, in particular, arginine, proline, glycine, valine and lisine.

The tissue prosthesis subject of the present invention may advantageously be used to repair any break in human or animal tissues, in particular incisional, umbilical or inguinal hernias, whether congenital or acquired; lesions to tendons or ligaments; skin lesions in general including diabetic ulcers; lesions to the meniscus or connective tissues in general.

The tissue prosthesis subject of the present invention may be inserted in an intra-peritoneal, sub-peritoneal or extra-peritoneal position for hernias; in a sub-cutaneous, intra-cutaneous or extra-cutaneous position for breaks in the skin; in an intra- or peritendinous or intra- or periligamentous position for breaks in tendons or ligaments.

Naturally, the realisation details and practical forms of the prosthesis can be widely varied from what is described and illustrated here, without leaving the sphere of protection of the present invention, as is defined by the attached claims.

## Claims

1. Tissue prosthesis (1) to repair tissues comprising a first layer (2) and a second layer (3), said layers extending in a substantially coplanar fashion with a peripheral zone (90) in which said layers are distanced from one another, said peripheral space (90) surrounding a central area (95) in which said layers are adjacent to one another, **characterised in that** in said central area (95) there is at least one zone (91) in which said layers are distanced from one another.

2. Tissue prosthesis according to claim 1, **characterised in that** said at least one zone (91) in which said layers are distanced from one another presents a spoke-like configuration.

3. Tissue prosthesis according to claim 1 or claim 2, **characterised in that** said at least one zone (91) in which said layers are distanced from one another presents a ring-shaped configuration.

4. Tissue prosthesis according to any one of the above claims, **characterised in that** said at least one zone (91) in which said layers are distanced from one another presents a grid configuration.

5. Tissue prosthesis according to any one of the above claims, **characterised in that** within said zones (90, 91) in which said layers are distanced from one another, suture anchors are present.

6. Tissue prosthesis according to any one of the above claims, **characterised in that** within said zones (90, 91) in which said layers are distanced from one another, in alternative or in association the following are present:
i) tissue islands;
ii) differentiated or stem cells;
iii) cell cultures, in particular cultures of fibroblasts;
iv) drugs; or
v) appropriate devices to improve healing of the tissue to be repaired.

7. Tissue prosthesis according to claim 6, **characterised in that** said drugs are NO donors.

8. Tissue prosthesis according to claim 7, **characterised in that** said drugs are selected from the group comprising: S-nitrosothiols, detanonoate, SIN-1, molsidomine, isosorbide mono- and di-nitrate, eritrile and pentaeritrile tetranitrate, tenitramine and, preferably, glyceryl trinitrate.

9. Tissue prosthesis according to claim 6, **characterised in that** said devices appropriate to improve healing of the tissue to be repaired are chemotactic substances for fibroblasts.

10. Tissue prosthesis according to claim 9, **characterised in that** said chemotactic substances are selected from the group comprising: elastin, amino acids, polyamino acid complexes, hyaluronic acid.

11. Tissue prosthesis according to claim 10, **characterised in that** said amino acids are selected from the group comprising: arginine, proline, glycine, valine and lisine.
